# EUROPEAN PATENT APPLICATION

(11) **EP 1 671 645 A1**
(43) Date of publication of application: **21.06.2006**
(21) Application number: 04772922.3
(22) Date of filing: 03.09.2004
(51) Int. Cl.: A61K 39/395, A61P 35/00, G01N 33/574, G01N 33/543, C07K 16/18

(54) **REMEDY AND DETECTION DRUG FOR BILE DUCT CANCER**

(30) Priority: 04.09.2003 JP 2003313187
(71) Applicant: CHUGAI SEIYAKU KABUSHIKI KAISHA, Tokyo 115 (JP); Perseus Proteomics Inc., Tokyo 151-0064 (JP); Aburatani, Hiroyuki, Musashino-shi, Tokyo 180-0003 (JP)
(72) Inventor: ABURATANI, Hiroyuki, Tokyo 1530041 (JP); FUKAYAMA, Masahisa, Tokyo 1130033 (JP); YAMAUCHI, Naoko, Tokyo 1130033 (JP); SUZUKI, Masami., Chugai Seiyaku K.K., Gotenba-shi, Shizuoka 4120038 (JP); KATO, Atsuhiko, Chugai Seiyaku K.K., Gotenba-shi, Shizuoka 4120038 (JP); TSUCHIYA, Masayuki, Chugai Seiyaku K.K., Gotenba-shi, Shizuoka 4120038 (JP)
(74) Representative: Woods, Geoffrey Corlett
(86) International application number: PCT/JP2004/013183
(87) International publication number: WO 2005/023301

(57) **Abstract**

Disclosed is a cholangiocarcinoma cell growth inhibitor comprising an anti-glypican-3 antibody as an active ingredient. Preferably, the anti-glypican-3 antibody has a cytotoxic activity such as an antibody-dependent cytotoxic (ADCC) activity and a complement-dependent cytotoxic (CDC) activity. Also disclosed is a diagnostic agent for diagnosis of cholangiocarcinoma comprising an anti-glypican-3 antibody.

## Description

### TECHNICAL FIELD

The present invention relates to a cholangiocarcinoma cell growth inhibitor and a diagnostic agent for cholangiocarcinoma that comprise an anti-glypican-3 antibody.

### BACKGROUND ART

The presence of a glypican family has been reported as a new family of heparan sulfate proteoglycan existing on the surface of cells. Up to date, it has been reported that a glypican family includes five glypican members (glypican-1, glypican-2, glypican-3, glypican-4, glypican-5). The members of this family have a core protein of the same size (about 60 kDa) and share a specific and well-conserved cysteine sequence, and they bind to the membrane of a cell via a glycosylphosphatidylinositol (GPI) anchor.

Dally (division abnormally delayed) gene has been identified from a genetic screening of a Drosophila melanogaster mutant characterized by an abnormal cell division pattern in the development of the central nervous system. It has been known that the cDNA of dally represents an open reading frame (ORF) that codes for the product showing a sequence homology (24 to 25 % homology) to a membrane-type proteoglycan (GRIPs) of a vertebrate that has all characteristics of glypican. Afterwards, it has been suggested that dally has a function of controlling the mechanism of a dpp (decapentaplegia) receptor, suggesting that glypican may possibly control the signal transmission of TGF and BMP in mammals. Specifically, it has been suggested that glypican may function as a common receptor for several heparin-binding growth factors (e.g., EGF, PDGF, BMP2, FGF).

Glypican-3 was separated as a developmentally controlled transcription product from rat small intestines (Filmus, J., Church, J. G., and Buick, R. N. (1988), *Mol. Cell Biol., 8,* 4243-4249), and thereafter it was identified as OCT-5, a GPI-binding heparan sulfate proteoglycan, that has a core protein having a molecular weight of 69 kDa (Filmus, J., Shi, W., Wong, Z.M., and Wong, M. J. (1995), *Biohcem.* J., 311, 561-565). Also in humans, a gene coding for glypican-3 has been isolated as MXR-7 from a human stomach cancer cell line (Hermann Lage, et al. , Gene 188 (1997), 151-156) . It has been reported that glypican-3 forms a protein-protein complex with insulin-like growth factor-2, and it controls the activity of the growth factor (Pilia, G., et al., (1996), *Nat.* Genet., 12, 241-247). This report suggests that glypican-3 does not always interact with the growth factor via a heparan sulfate chain.

A potential use of glypican-3 as a hepatocarcinoma marker has been suggested (Hey-Chi Hsu, et al., Cancer *Research* 57, 5179-5184 (1997)). Also it has been suggested that glypican may function as an endostatin receptor that could act as a vascularization inhibitor *(Molecular Cell* (2001), 7, 811-822). Further, an anti-glypican-3 antibody was reported to have a cytotoxic activity against cancer cells such as hepatocarcinoma cells (WO03/00883, WO04/22739). However, there is no report concerning expression of glypican-3 in cholangiocarcinoma cells.

An object of the present invention is to provide a cholangiocarcinoma cell growth inhibitor and a diagnostic agent for cholangiocarcinoma that comprise an anti-glypican-3 antibody.

### DISCLOSURE OF THE INVENTION

The present inventors have assiduously studied and have found that glypican-3 is highly expressed in cholangiocarcinoma cells and that an anti-glypican-3 antibody may be effective as a cholangiocarcinoma cell growth inhibitor based on its cytotoxic activity and as a diagnostic agent for cholangiocarcinoma.

An anti-glypican-3 antibody will exhibit a cell growth-inhibiting activity when a cytotoxic substance such as a radioactive isotope, a chemical therapeutical agent or a bacterial toxin is attached to the antibody.

Further, it has been found that glypican-3 is localized in the inner lumen of a normal bile duct, while its distribution is altered in cholangiocarcinoma where it is localized in the entire periphery of the cell membrane. This finding indicates that an anti-glypican-3 antibody may readily reach cholangiocarcinoma cells but hardly reaches normal bile duct cells where the protein is expressed in the bile duct inner lumen. Accordingly, an anti-glypican-3 antibody is expected to be especially effective for treatment of cholangiocarcinoma.

Specifically, the invention provides the following:
(1) A cholangiocarcinoma cell growth inhibitor comprising an anti-glypican-3 antibody;
(2) The cholangiocarcinoma cell growth inhibitor of (1), wherein the anti-glypican-3 antibody has a cytotoxic activity;
(3) The cholangiocarcinoma cell growth inhibitor of (2), wherein the cytotoxic activity is an antibody-dependent cytotoxic (ADCC) activity or a complement-dependent cytotoxic (CDC) activity;
(4) The cholangiocarcinoma cell growth inhibitor of (1), wherein the anti-glypican-3 antibody is an antibody having a cytotoxic substance attached thereto;
(5) The cholangiocarcinoma cell growth inhibitor of (1) to (4), wherein the antibody is a monoclonal antibody;
(6) The cholangiocarcinoma cell growth inhibitor of (1) to (4), wherein the antibody is a humanized or chimera antibody;
(7) A diagnostic agent for cholangiocarcinoma comprising an anti-glypican-3 antibody;
(8) The diagnostic agent for cholangiocarcinoma of (7) comprising an anti-glypican-3 antibody immobilized on a support and an antibody labeled with a labeling substance;
(9) The diagnostic agent for cholangiocarcinoma of (7) or (8), wherein the antibody is a monoclonal antibody;
(10) A diagnostic kit for diagnosis of cholangiocarcinoma comprising an anti-glypican-3 antibody;
(11) The diagnostic kit for diagnosis of cholangiocarcinoma of (10) comprising an anti-glypican-3 antibody immobilized on a support and an antibody labeled with a labeling substance;
(12) A method for inhibiting the growth of cholangiocarcinoma cells comprising administering an anti-glypican-3 antibody to a patient suffered from cholangiocarcinoma;
(13) A method for diagnosing cholangiocarcinoma comprising detecting a glypican-3 protein in an assay sample.

According to the invention, there is provided a therapeutic agent for cholangiocarcinoma comprising an anti-glypican-3 antibody as an active ingredient. As demonstrated in the Examples below, GPC3 is expressed at a high level in cholangiocarcinoma cells, thus an antibody against the protein is expected to have an anti-carcinoma effect on cholangiocarcinoma cells. In addition, it is considered that the antibody may readily reach the tumor cells that express the protein uniformly in the cell membrane, while the antibody could hardly reach the normal bile duct cells that express the protein in the bile duct inner lumen. Accordingly, an antibody against the protein could represent a therapeutic agent specific to cholangiocarcinoma which will not damage normal bile duct cells.

### BRIEF DESCRIPTION OF THE DRAWINGS

Fig. 1 is a photograph of immunostaining of cholangiocarcinoma cells with an anti-GPC-3 antibody.
Fig. 2 is a photograph of immunostaining of normal cells with an anti-GPC-3 antibody.

### DETAILED DESCRIPTION OF THE INVENTION

The invention is related to a cholangiocarcinoma cell growth inhibitor and a diagnostic agent for cholangiocarcinoma that comprise an anti-glypican-3 antibody.

The anti-glypican-3 antibody of the invention includes known antibodies such as humanized antibodies, human antibodies (WO96/33735), chimera antibodies (JP-A 4-228089), and mouse antibodies. The antibody may be a polyclonal antibody but is preferably a monoclonal antibody.

The anti-glypican-3 antibody for use in the invention is not specifically limited in terms of its origin, type (monoclonal, polyclonal) and form, so far as it has an ability of binding to glypican-3.

The cholangiocarcinoma cell growth inhibitor of the invention may be any types of agent that comprises an anti-glypican-3 antibody as an active ingredient.

It has been already reported that an anti-glypican-3 antibody has a cytotoxic activity to the cells that express glypican-3, and is therefore useful as an anti-cancer agent (WO03/00883, WO04/22739).

### 1. Anti-glypican-3 antibody

The anti-glypican-3 antibody for use in the invention may be obtained as a polyclonal or monoclonal antibody in any known method. As the anti-glypican-3 antibody for use in the invention, especially preferred is a monoclonal antibody derived from a mammal. The monoclonal antibody derived from a mammal includes those produced by hybridoma, and those produced by a host transformed with an expression vector containing the antibody gene by means of a genetic engineering technique. The antibody will bind to glypican-3 and inhibit cell growth.

Such an antibody may be obtained by any method known in the art.

### 2. Antibody-producing hybridoma

Essentially according to a known technique, a hybridoma producing a monoclonal antibody can be produced in the manner described below. Cells are immunized with glypican-3 as a sensitizing antigen in a standard immunization method, and the resulting immune cells are fused with known parent cells according to a standard cell fusion method, and the resulting fused cells are screened for monoclonal antibody-producing cells according to a standard screening method.

Specifically, a monoclonal antibody may be produced as follows.

First, human glypican-3 to be used as a sensitizing antigen for raising an antibody is prepared by expressing a glypican-3 (MXR7) according to the gene/amino acid sequence disclosed in Lage, H., et al., Gene 188 (1997), 151-156. Specifically, a gene sequence that codes for glypican-3 is inserted into a known expression vector system, suitable host cells are transformed with the vector, and then the intended human glypican-3 protein is isolated and purified from the host cells or the culture supernatant thereof, according to a known method.

Next, the purified glypican-3 protein is used as a sensitizing antigen. Alternatively, a partial peptide of glypican 3 may be used as a sensitizing antigen. Such a partial peptide may be obtained through chemical synthesis based on the amino acid sequence of human glypican-3.

The anti-glypican-3 antibody has a cell growth inhibiting activity based on its ADCC activity, CDC activity and growth factor activity-inhibiting activity, and it can inhibit cell growth if a cytotoxic substance is attached, such as a radioactive isotope, a chemical therapeutical agent or a bacterial toxin. The anti-glypican-3 antibody of the invention may recognize any epitope within a glypican-3 molecule. Accordingly, the antigen to be used for producing the anti-glypican-3 antibody of the invention may be any fragment that represents an epitope existing on a glypican-3 molecule.

The mammalian animal to be immunized with a sensitizing antigen is not specifically limited, but is preferably selected in consideration of the compatibility with the parent cells used for the cell fusion. Commonly used mammals are rodents such as mouse, rat and hamster, as well as rabbit and monkey.

Such an animal may be immunized with a sensitizing antigen by any known method. For example, a sensitizing antigen is intraabdominally or subcutaneously injected into an animal. Specifically, a sensitizing antigen is diluted and suspended at a suitable concentration in PBS (phosphate-buffered saline) or physiological saline, and optionally mixed with a suitable amount of a conventional adjuvant such as a Freund's complete adjuvant, and then emulsified. The resulting emulsion is administered to an animal for several times in 4-21 day intervals. If desired, a suitable carrier may be used in immunization with a sensitizing antigen.

An animal is immunized in the manner as above, and after the increase in the desired antibody level in the serum, the immune cells are collected from the animal and subjected to cell fusion. Preferably, the immune cell is a spleen cell.

The parent cell to be fused with the immune cell may be a mammalian myeloma cell. The myeloma cell conveniently used for cell fusion includes various known cell lines, for example, P3 (P3x63Ag8.653) (*J. Immunol.* (1979) 123, 1548-1550), P3x63Ag8U.1 (Current *Topics in Microbiology* and *Immunology* (1978) 81, 1-7), NS-1 (Kohler, G. and Milstein, C., *Eur. J. Immunol.* (1976) 6, 511-519), MPC-11 (Margulies, D. H., et al., *Cell* (1976) 8, 405-415), SP2/0 (Shulman, M., et al., *Nature* (1978) 276, 269-270), FO (deSt. Groth, S. F. , et al. , *J. Immunol. Methods* (1980) 35, 1-21), S194 (Trowbridge, I. S. J*. , Exp. Med.* (1978) 148, 313-323), R210 (Galfre, G., et al. , *Nature* (1979) 277, 131-133).

The cell fusion of the immune cell with such a myeloma cell may be effected essentially according to known methods, for example, Kohler and Milstein's method (Kohler, G., and Milstein, C., *Methods Enzymol.* (1981) 73, 3-46).

More specifically, the cell fusion may be effected, for example, in a common nutrient medium in the presence of a cell fusion stimulator. The cell fusion stimulator used in the invention includes, for example, polyethylene glycol (PEG) and Sendai virus (HVJ). Optionally an auxiliary agent such as dimethylsulfoxide may be added to the medium to increase the fusion efficiency.

The proportion of the number of immune cells to the number of myeloma cells may be set to any desired ratio. For example, it is desirable that the number of the immune cells is from 1 to 10 times that of the myeloma cells. The culture medium suitable for use in the cell fusion may include, for example, RPMI1640 medium or MEM medium favorable for the growth of the above-mentioned myeloma cell lines, as well as other culture media generally used for cultivating cells of this type. In addition, a serum complement such as fetal calf serum (FCS) may be added to the culture medium.

For the cell fusion, the above-mentioned immune cells and myeloma cells are well mixed in a predetermined ratio in a culture medium, and a PEG solution (for example, with an average molecular weight of from about 1000 to about 6000) previously heated at about 37°C is added generally at a concentration of from 30 to 60 % (w/v), and mixed to obtain a hybridoma. Subsequently, a suitable medium is added and centrifuged to remove the supernatant. This process is repeated to remove the cell fusion agent and other materials unfavorable to the growth of the hybridoma.

The hybridoma thus obtained may be selected through cultivation in a standard selective culture medium, for example, HAT medium (a medium containing hypoxanthine, aminopterin and thymidine) . The cultivation in the HAT medium is continued for a period of time sufficient for the death of the cells other than the intended hybridoma (non-fused cells). This step generally takes a few days to a few weeks. Next, the hybridoma is screened and monocloned according to a standard limiting dilution method to isolate those clones capable of producing the intended antibody.

Beside the above-mentioned method of immunizing a non-human animal with an antigen to obtain the intended hybridoma, another method may also be employed, where a human lymphocyte is sensitized with glypican-3 in vitro, and the sensitized lymphocyte is fused with human myeloma cells capable of dividing permanently, whereby a desired human antibody having a binding activity to glypican-3 can be obtained(see JP-B 1-59878). Alternatively, the antigen glypican-3 is administered to a transgenic animal having all repertories of human antibody genes to obtain a cell producing an anti-glypican-3 antibody. Then the cell is immortalized and a human antibody against glypican-3 may be obtained from the immortalized cells (see International Publication Nos. WO94/25585, WO93/12227, WO92/03918, WO94/02602).

The monoclonal antibody-producing hybridoma thus obtained may be subcultured in a conventional culture medium, and stored in liquid nitrogen for a long period of time.

To produce a monoclonal antibody from the hybridoma, the hybridoma may be cultivated by a conventional method and the culture supernatant may be collected; or the hybridoma may be introduced into and grown in an animal compatible with the hybridoma, and the ascites containing the antibody may be collected from the animal. The former method is suitable for obtaining antibodies in high purity; and the latter is suitable for mass-production of antibodies.

### 3. Recombinant antibody

In the invention, a recombinant monoclonal antibody can be obtained according to a genetic recombination technique as follows: An antibody gene is cloned from the hybridoma and inserted into a suitable vector, and the vector is introduced into a host to produce the intended recombinant monoclonal antibody (for example, see Vandamme, A. M., et al., *Eur. J. Biochem.* (1990) 192, 767-775, 1990).

Specifically, mRNA that codes for the variable (V) region of the anti-glypican-3 antibody is isolated from the hybridoma producing an anti-glypican-3 antibody. The isolation of mRNA may be effected according to a known method. For example, a full-length RNA is prepared through guanidine ultra-centrifugation (Chirgwin, J. M., et al., *Biochemistry.* (1979) 18, 5294-5299) or AGPC (Chomczynski, P., et al., *Anal. Biochem.* (1987) 162, 156-159), and the intendedmRNA is prepared using mRNA Purification Kit (by Pharmacia) or the like. Alternatively, the mRNA may be directly prepared using Quick-Prep mRNA Purification Kit (by Pharmacia).

cDNA for the antibody V-region is synthesized from the thus obtained mRNA using reverse transcriptase. The cDNA synthesis may be effected using AMV Reverse Transcriptase First-Strand cDNA Synthesis Kit (by Seikagaku Kogyo) or the like. cDNA may also be synthesized and amplified by the 5' -RACE method and PCR with 5' -Ampli FINDER RACE Kit (by Clontech) (Frohman, M. A. , et al., *Proc. Natl. Acad. Sci . USA* (1988) 85, 8998-9002; Elyavsky, A., et al. , *Nucleic Acids Res.* (1989) 17, 2919-2932).

The intended DNA fragment is purified from the PCR product, and linked to a vector DNA. Then the recombinant vector is introduced into *E*. *coli* or the like, and colonies are selected, whereby the intended recombinant vector is prepared. Then, the nucleotide sequence of the DNA is confirmed in a known method, for example, according to a dideoxynucleotide chain termination method.

Once the intended DNA coding for an anti-glypican-3 antibody V-region is obtained, it is inserted into an expression vector that contains DNA coding for a suitable antibody constant region (C region).

To produce the anti-glypican-3 antibody to be used in the invention, essentially an antibody gene is inserted into an expression vector in such a manner that it may be expressed under the control of the expression control region, for example, an enhancer or a promoter. Next, a host cell is transformed with the expression vector, and the antibody is expressed in the cell.

For the antibody gene expression, DNAs that code for the antibody heavy chain (H-chain) and light chain (L-chain) may be separately inserted into expression vectors and co-transform a host cell; or DNAs that code for the H-chain and the L-chain may be inserted into a single expression vector and transform a host cell (see WO94/11523).

In addition to the above-mentioned host cells , transgenic animals may also be used for producing the recombinant antibody, For example, an antibody gene is inserted into the middle of a gene that codes for a protein intrinsically produced in a milk (e.g., goat-β casein) to prepare a fused gene. A DNA fragment that contains the fused gene with the antibody gene is injected into a goat embryo, and the embryo is introduced into a female goat. The intended antibody is obtained from the milk produced by a transgenic goat born from the embryo-received goat or by its posterity. For increasing the amount of the milk containing the intended antibody produced by the transgenic goat, a suitable hormone may be administered to the transgenic goat (Ebert, K. M., et al., *Bio*/*Technology* (1994) 12, 699-702).

### 4. Modified antibody

In the invention, any other recombinant antibodies other than those described above may also be used, such as a chimera antibody and a humanized antibody that has been artificially modified for the purpose of lowering the hetero-antigenicity to human. Such a modified antibody may be produced according to a known method.

A chimera antibody may be obtained as follows: DNA encoding the antibody V-region obtained as above is linked to DNA encoding a human antibody C-region, and inserted into an expression vector. The resulting vector is introduced into a host to produce the chimera antibody. According to this known method, a chimera antibody useful in the invention may be obtained.

A humanized antibody, also referred to as a reshaped human antibody, is constructed by grafting the complementarity-determining region (CDR) of an antibody from a non-human mammal (e.g. a mouse antibody) into the complementarity-determining region of a human antibody. A general genetic recombination method for producing a humanized antibody is known in the art (see European Patent Application No. EP 125023; WO96/02576).

Specifically, a DNA sequence designed to link CDR of a mouse antibody to the framework region (FR) of a human antibody is synthesized through PCR using as primers several oligonucleotides constructed so as to have overlapping portions at the terminal region of both CDR and FR (see the method described in WO98/13388).

The framework region of a human antibody to be linked to CDR is selected such that the complementarity-determining region may form an appropriate antigen-biding site. If desired, the amino acid residues in the framework region of the variable region of the antibody may be substituted so that the complementarity-determining region of the reshaped human antibody may form a suitable antigen-binding site (Sato, K. et al., *Cancer Res.* (1993) 53, 851-856).

Generally, C-regions derived from a human antibody is used as the C-region of chimera antibody and humanized antibody. For example, Cγ1, Cγ2, Cγ3 , Cγ4 may be used for the H-chain, and Cκ and Cλ may be used for the L-chain. The human antibody C-region may be modified in order to improve the stability of the antibody and its production process.

A chimera antibody comprises a variable region of an antibody derived from a non-human mammal, and a constant region of a human antibody. On the other hand, humanized antibody comprises the complementarity-determining region of an antibody derived from a non-human mammal, and a framework region and a constant region derived from a human antibody. Since a humanized antibody should have lower antigenicity in human bodies, the humanized antibody is useful as an active ingredient of the therapeutic agent of the invention.

### 5. Modified antibodies

The antibody for use in the invention is not limited to the entire molecule of an antibody, but may be a fragment of an antibody or its modified derivative, including a divalent antibody and a monovalent antibody, so far as it has an ability to bind to glypican-3 and inhibit cell growth,. For example, an antibody fragment includes Fab, F(ab')2, Fv, Fab/c having one Fab and a complete Fc, single chain Fv (scFv) with an H-chain or L-chain Fv linked with a suitable linker, and a diabody. Such an antibody fragment may be prepared by processing an antibody with an enzyme such as papain or pepsin. Alternatively, a gene coding for such an antibody fragment is constructed, introduced into an expression vector, and then expressed in a suitable host cell (for example, see Co. M. S., et al., J. *Immunol.* (1994) 152,2968-2976; Better, M. & Horwitz, A. H., *Methods in Enzymology* (1989) 178, 476-496; Academic Press, Inc., Pleuckthun, A. & Skerra, a., *Methods in Enzymology* (1989) 178, 476-496; Academic Press, Inc., Lamoyi, E., *Methods in Enzymology* (1989) 121, 652-663; Rousseaux, J., et al., *Methods in Enzymology* (1989) 121, 663-669; Bird, R. E. , et al. , *TIBTECH* (1991) 9, 132-137).

scFv is obtained by linking the H-chain V-region and the L-chain V-region of an antibody. In the scFv, the H-chain V-region and the L-chain V-region are linked via a linker, preferably a peptide linker (Huston, J. S. , et al. , *Proc. Natl. Acad. Sci., USA* (1988) 85, 5879-5883). The H-chain V-region and the L-chain V-region in scFv may be derived from any antibodies described above. The peptide linker that links the V-regions may comprise any linear peptide having from 12 to 19 amino acid residues.

DNA encoding scFv may be obtained as follows. A DNA fragment is amplified by PCR using as a template a DNA portion that codes for the entire or the desired amino acid sequence of the H-chain or the H-chain V-region of an antibody, and a DNA coding for the L-chain or the L-chain V-region thereof, and a primer pair that defines the two ends of the sequence. Then the fragment is further amplified by the use of a combination of DNA that codes for the peptide linker portion and a primer pair that defines both ends to be linked to the H-chain and the L-chain.

Once the scFv-encoding DNA is constructed, an expression vector containing the DNA, and a host transformed with the expression vector may be obtained by any standard method. The scFv can be produced in such a host by any standard method.

These antibody fragments can be produced in a host by obtaining the gene in the same manner as above and allowing it to be expressed. The "antibody" as used herein also includes such antibody fragments.

A modified anti-glypican antibody conjugated with a various molecule, for example, a cytotoxic substance or polyethylene glycol (PEG) may also be used in the invention. The cytotoxic substance includes, for example, radioactive isotope, chemical therapeutical agent and bacterial toxin. The "antibody" in the invention includes such a modified antibody conjugated with other substances. The modified antibody can be obtained by chemically modifying the antibody obtained as above. The antibody modification methodology has been already established in the art.

The antibody for use in the invention may also be a bispecific antibody. The bispecific antibody may have antigen-binding sites capable of recognizing different epitopes on a glypican-3 molecule, or may have one antigen-binding site recognizing glypican-3 and another antigen-binding site recognizing a cytotoxic substance such as chemotherapeutical agent, a bacterial toxin or a radioactive substance. In this case, a cytotoxic substance will directly act on glypican-3-expressing cells to specifically damage the tumor cells, whereby the tumor cells may be prevented from growing. The bispecific antibody can be constructed by combining the HL pair of two different types of antibodies, or by fusing different hybridoma cells each producing a different monoclonal antibody to provide a fusion cell producing the bispecific antibody. In addition, the bispecific antibody may also be constructed according to a genetic engineering method.

For enhancing the cytotoxic activity of the antibody, the sugar chain of the antibody may be modified. Technique for modifying sugar chains of an antibody are already known (for example, WO00/61739, WO02/31140).

### 6. Expression and production of recombinant antibody or modified antibody

The antibody gene constructed in the manner as above may be expressed in a known method to obtain an antibody. In the case of mammalian cells, a conventional promoter, an antibody gene to be expressed, and a poly-A signal at 3' -downstream may be operably linked to allow for antibody expression. For example, the promoter/enhancer may include a human cytomegalovirus immediate early promoter/enhancer.

An additional promoter/enhancer used for antibody expression in the invention includes viral promoter/enhancer of retrovirus, polioma virus, adenovirus, simian virus 40 (SV40), as well as a promoter/enhancer derived from a mammalian cell such as human elongation factor 1α (HEF1α).

The intended gene may be readily expressed according to the Mulligan's method (Nature (1979) 277, 108) when SV40 promoter/enhancer is used; or the Mizushima's method (*Nucleic Acids Res.* (1990) 18, 5322) when HEF1α promoter/enhancer is used.

In the case of E. coli, a conventional promoter, a signal sequence for antibody secretion and an antigen gene to be expressed are operably linked together, and is expressed in the cells. The promoter includes, for example, lacZ promoter, and araB promoter. The intended gene may be expressed according to the Ward's method (Nature (1098) 341, 544-546; *FASEB J.* (1992), 6, 2422-2427) when lacZ promoter is used, or the Better's method (Science (1998) 240, 1041-1043) when araB promoter is used.

A signal sequence such as pelB signal sequence (Lei, S. P., et al., *J*. *Bacteriol.* (1987) 169, 4379) may be used for producing antibody into the periplasm of *E*. *coli.* After the antibody produced in the periplasm is separated, the antibody is suitably refolded and used.

The replication origin used in the expression vector may include those derived from SV40, polioma virus, adenovirus or bovine papilloma virus (BPV). To increase the number of gene copies in the host cells, the expression vector may contain as a selective marker aminoglycoside transferase (APH) gene, thymidine kinase (TK) gene, E. coli xanthine-guanine-phosphoribosyl transferase (Ecogpt) gene, dihydrofolic acid reductase (dhfr) gene or the like.

Any expression system may be used for producing the antibody for use in the invention, for example, an eukaryotic cell line or a prokaryotic cell line. The eukaryotic cells include established animals cell lines, for example, mammalian cell lines or insect cell lines, as well as filamentous fungal cells and yeast cells. The prokaryotic cells include bacterial cells such as *E*. *coli* cells.

Preferably, the antibody for use in the invention is expressed in mammalian cells, such as CHO, COS, myeloma, BHK, Vero, and Hela cells.

Next, the transformed host cells are grown in vitro or in vivo to produce the intended antibody. The host cell cultivation may be effected according to a known method. For example, DMEM, MEM, RPMI1640 and IMDM may be used for a culture medium, and a serum complement such as fetal calf serum (FCS) may be added to the culture medium.

### 7. Separation and purification of antibody

The antibody expressed and produced in the manner as above may be isolated from the cells or the host animal and purified to homogeneity. An affinity column may be used for separation and purification of the antibody for use in the invention. For example, a column based on the Protein A column includes Hyper D, POROS, Sepharose F.F. (by Pharmacia) . In addition, any other methods generally used for protein separation and purification may be employed in the invention. Apart from the above-mentioned affinity column, any other means, such as chromatography column, filter, ultrafiltration, salting-out and dialysis may be suitably selected and combined for use in antibody separation and purification *(Antibodies A Laboratory Manual,* Ed. Harlow, David Lane, Cold Spring Harbor Laboratory, 1988).

### 8. Measurement of antibody activity

The antibody for use in the invention may be analyzed for its antigen-binding activity in any known methods *(Antibodies A Laboratory Manual,* Ed. Harlow, David Lane, Cold Spring Harbor Laboratory, 1988) and its ligand receptor-binding inhibiting activity (Harada, A., et al., International Immunology (1998) 5, 681-690).

The antigen-binding activity of the anti-glypican-3 antibody for use in the invention may be determined by ELISA (enzyme-linked immunosorbent assay), EIA (enzyme immunoassay), RIA (radioimmunoassay) or immunofluorescence. For example, enzyme immunoassay may be carried out as follows. A sample containing an anti-glypican-3 antibody, for example, a culture supernatant or a purified antibody from the cells producing an anti-glypican-3 antibody, is added to a plate coated with glypican-3. A secondary antibody labeled with an enzyme such as alkali phosphatase is added, and the plate is incubated and washed. Then an enzyme substrate such as p-nitrophenylphosphate is added, and the absorbance of the plate is measured to determine the antigen-binding activity of the antibody.

### 9. Cytotoxic activity

The antibody for use in the invention has a cytotoxic activity, i.e. an ADCC activity and/or CDC activity.

The ADCC activity of the antibody may be determined by mixing an effector cell, a target cell and an anti-glypican-3 antibody and measuring ADCC activity of the resulting mixture. The effector cell includes, for example, mouse myeloma cells or monocytes separated from human peripheral blood or bone marrow. The target cell includes, human established cell lines such as human liver cancer cell line HuH-7. The target cells are previously labeled with ⁵¹Cr, and incubated with the anti-glypican-3 antibody, then the effector cells are added at a suitable ratio to the target cells. After incubation, the supernatant is collected, and the radioactivity in the supernatant is counted to determine the ADCC activity of the antibody.

The CDC activity of the antibody may be determined as follows. The above-mentioned labeled target cells are mixed with an anti-glypican-3 antibody, and then a complement is added. After incubation, the radioactivity in the supernatant is counted to determine the CDC activity of the antibody

The antibody must have an Fc portion to exhibit its cytotoxic activity. Where the anti-glypican-3 antibody is used in the invention as a cell growth inhibitor based on the cytotoxic activity of the antibody, it must contain the Fc portion.

### 10. Other glypican-3 inhibiting substance

The cholangiocarcinoma cell growth inhibitor of the invention may contain a substance capable of binding to glypican 3, other than the anti-glypican-3 antibody. For example, the inhibitor may contain a cytotoxic compound attached to a substance capable of binding to glypican-3.

### 11. Administration method and pharmaceutical preparation

The cholangiocarcinoma cell growth inhibitor of the invention may be used for treating or curing diseases caused by abnormal growth of cholangiocarcinoma cells, especially cholangiocarcinoma.

The effective dose of the inhibitor may be selected from a range of from 0.001 mg to 1000 mg per kg body weight or from 0.01 to 100000 mg/body of a patient. However, the dose of the agent comprising the anti-glypican-3 antibody of the invention is not limited to the above range.

The agent of the invention may be administered to the patient anytime before or after the development of the clinical symptom of the disease.

The agent of the invention may be administered once to three times a day for one to 7 days a week.

In general, the agent of the invention may be administered parenterally, for example, through injection (e.g., subcutaneous injection, intravenous injection, intramuscular injection, and intraabdominal injection) or through any other route of administration , for example, percutaneous, mucosal, nasal, transpulmonary or oral administration. However, mode of administration of the agent of the invention should not be limited to the dose and administration method described above.

The agent of the invention comprising an anti-glypican-3 antibody as an active ingredient may be formulated according to any standard method (*Remington's Pharmaceutical Science,* latest edition, Mark Publishing Company, Easton, USA), and may contain any pharmaceutically-acceptable carriers and additives.

Examples of the carriers and pharmaceutical additives include water, pharmaceutically-acceptable organic solvents, collagen, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, agar, polyethylene glycol, diglycerin, glycerin, propylene glycol, vaseline, paraffin, stearyl alcohol, stearic acid, human serum albumin (HSA), mannitol, sorbitol, lactose, and surfactants acceptable as pharmaceutical additives.

The additives may be suitably selected from, but not limited to, the above list, and used alone or in combination depending on the preparation forms of the agent of the invention. For example, an agent in the form of an injection preparation may be formulated as follows. A purified anti-glypican-3 antibody is dissolved in a solvent, for example, in a physiological saline, buffer or glucose solution, and an adsorption inhibitor such as Tween 80, Tween 20, gelatin or human serum albumin is added. The pharmaceutical composition may be freeze-dried for a preparation form to be dissolved in a solvent just before use. The freeze-dried composition may be prepared using an excipient, such as glycoalcohol and saccharide, e.g., mannitol and glucose.

### 12. Diagnosis of cholangiocarcinoma

The invention provides a method for diagnosing (or detecting) cancer by detecting glypican-3 in an assay sample.

The detection includes quantitative or non-quantitative detection. For example, the non-quantitative detection includes analyzing a sample for the presence or absence of GPC3 protein in the sample; analyzing a sample for the presence or absence of GPC3 protein in an amount above the predetermined level; comparing the amount of GPC3 protein in a sample with another sample (for example, control sample). The quantitative detection includes determining the concentration of GPC3 protein in a sample and determining the amount of GPC3 protein in a sample.

The assay sample is not limited as long as it may contain GPC3 protein, but is preferably those collected from a living body such as a mammal, more preferably those collected from a human. Examples of the assay sample include blood, interstitial fluid, plasma, extravascular fluid, cerebrospinal fluid, synovial fluid, pleural fluid, serum, lymphatic fluid, saliva and urine; it is preferably blood, serum, or plasma. In addition, samples derived from test samples, for example, culture medium of the cells collected from the body of an organism are also included in the assay sample analyzed by the invention.

Cancer to be diagnosed in the invention is cholangiocarcinoma.

Diagnosis of cholangiocarcinoma according to the invention may be carried out using the above-mentioned antibody. In the case that glypican-3 in an assay sample is detected according to a sandwich method, the anti-GPC3 antibody to be fixed on a support and the anti-GPC antibody to be labeled with a labeling substance may recognize the same epitope of GPC3 molecule, but preferably they recognize different epitopes.

GPC3 to be detected in the invention is not limited to a specific form, and it may be a full-length GPC3 or may be a fragment of a full-length GPC3. Where a GPC3 fragment is to be detected, it may be either an N-terminal fragment or a C-terminal fragment, but is preferably an N-terminal fragment (WO04/22739). The GPC3 protein may be a GPC3 protein having heparan sulfate added to the protein, or a GPC3 core protein.

The method of detecting GPC3 protein in an assay sample is not limited to a specific method, but preferably the protein is detected according to an immunological method using an anti-GPC3 antibody. The immunological method includes, for example, radioimmunoassay, enzyme immunoassay, fluoroimmunoassay, luminescent immunoassay, immunoprecipitation, immunonephelometry, Western blotting, immunostaining, and immunodiffusion; it is preferably enzyme immunoassay, and more preferably enzyme-linked immunosorbent assay (ELISA) (e.g., sandwich ELISA). ELISA and other immunological methods described above may be carried out in any manner known by those skilled in the art.

A detection method using an anti-GPC3 antibody generally comprises, for example, immobilizing an anti-GPC3 antibody on a support, adding an assay sample, incubating the sample to allow for the binding of the anti-GPC3 antibody to GPC3 protein, washing, and detecting the GPC3 protein bound to the support via the anti-GPC3 antibody, whereby the GPC3 protein in the assay sample may be detected.

The support to be used in the invention includes, for example, insoluble polysaccharides such as agarose, cellulose; synthetic resins such as silicone resin, polystyrene resin, polyacrylamide resin, nylon resin, polycarbonate resin; and insoluble supports such as glass. These supports may be used in the form of beads or plates. Such beads may be filled in a column for use in the invention. Plates may include a multi-well plate (e.g., 96-well plate), or a biosensor chip. The anti-GPC3 antibody may be bound to the support in any conventional manner such as chemical bonding or physical adsorption. Such supports are commercially-available.

The binding of the anti-GPC3 antibody to the GPC3 protein may be effected generally in a buffer. The buffer may include, for example, phosphate buffer, Tris buffer, citrate buffer, borate buffer, and carbonate buffer. The incubation may be carried out under conditions commonly used in the art, for example, at 4°C to room temperature for 1 hour to 24 hours. After the incubation, the support may be washed with any reagent as long as it does not interfere with the binding of the GPC3 protein to the anti-GPC3 antibody, for example, a buffer containing a surfactant such as Tween 20.

In the method of the invention for detecting GPC3 protein, a control sample may be included in addition to the assay sample to be detected for GPC3 protein. The control sample includes, for example, a negative control sample not containing GPC3 protein, and a positive control sample containing GPC3 protein. In this case, the GPC3 protein in the assay sample may be determined by comparing the result from the assay sample with the result from the GPC3 protein-free negative control sample or with the result from the GPC3 protein-containing positive control sample. A series of control samples having incremental protein concentrations are prepared, and the detection data from each control sample are plotted to prepare a standard calibration curve. The GPC3 protein in the assay sample may be quantitatively determined by comparing the data from the assay sample with the standard calibration curve.

In a preferred embodiment, GPC3 protein bound to the support via an anti-GPC3 antibody is detected using an anti-GPC3 antibody labeled with a labeling substance.

For example, an assay sample is contacted with an anti-GPC3 antibody immobilized on a support, washed, and then the GPC3 protein in the sample is detected by the use of a labeled antibody capable of specifically recognizing the protein.

The anti-GPC3 antibody may be labeled in any method generally known in the art. The labeling substance may be of any type of the substance known in the art, including, for example, fluorescent dye, enzyme, co-enzyme, chemical luminescent substance, and radioactive substance. Specific examples of the substance include radioisotopes (e.g. , ³²P , ¹⁴C, ¹²⁵I, ³H, ¹⁸³I), fluorescein , rhodamine, dansyl chloride, umbelliferone, luciferase, peroxidase, alkali phosphatase, β-galactosidase, β-glucosidase, horse radish peroxidase, glucoamylase, lysozyme, saccharide oxidase, microperoxidase, and biotin. When biotin is used as a labeling substance, it is desirable that a biotin-labeled antibody is added to the assay sample and then avidin conjugated with an enzyme such as alkali phosphatase is added. The labeling substance may be attached to the anti-GPC3 antibody by any known method, such as a glutaraldehyde method, a maleimide method, a pyridyl disulfide method or a periodic acid method.

Specifically, a solution containing an anti-GPC3 antibody is added to a support such as a plate to allow the anti-GPC3 antibody to be immobilized on the support. After the plate is washed, the plate is blocked with, for example, BSA, gelatin or albumin in order to prevent any non-specific binding of proteins. The plate is washed again, and an assay sample is added to the plate. After incubation, the plate is washed, and a labeled anti-GPC3 antibody is added. After appropriate incubation, the plate is washed, and the labeled-anti-GPC antibody remaining on the plate is detected. The detection may be effected in any method known by those skilled in the art. For example, where the antibody is labeled with a radioactive substance, the protein may be detected by liquid scintillation or RIA. Where the antibody is labeled with an enzyme, a substrate is added and the protein may be detected through the enzymatic change of the substrate, for example, by measuring coloration by an spectrophotometer. Specific examples of the substrate include diammonium 2,2-azinobis(3-ethylbenzothiazoline-6-sulfonate) (ABTS), 1,2-phenylenediamine (ortho-phenylenediamine), and 3,3',5,5'-tetramethylbenzidine (TME). When a fluorescent substance is used, the protein may be detected by the use of a fluorophotometer.

In an especially preferred embodiment of the method according to the invention, GPC3 protein is detected with a biotin-labeled anti-GPC3 antibody and avidin.

Specifically, a solution containing an anti-GPC3 antibody is added to a support such as a plate to allow the anti-GPC3 antibody to be immobilized on the support. After the plate is washed, the plate is blocked with, for example, BSA in order to prevent any non-specific binding of proteins. The plate is washed again, and an assay sample is added to the plate. After incubation, the plate is washed and a biotin-labeled anti-GPC3 antibody is added. After appropriate incubation, the plate is washed, and avidin attached to an enzyme such as alkali phosphatase or peroxidase is added. After incubation, the plate is washed, and a substrate corresponding to the enzyme attached to avidin is added, and the GPC3 protein is detected based on the enzymatic change of the substrate indicative of the presence of the protein.

In another embodiment of the method according to the invention, GPC3 protein is detected using a primary antibody capable of specifically recognizing GPC3 protein and a secondary antibody capable of specifically recognizing the primary antibody.

For example, an assay sample is contacted with an anti-GPC antibody immobilized on a support, the support is incubated and washed, and then the bound GPC3 protein is detected with the primary anti-GPC3 antibody and the secondary antibody capable of specifically recognizing the primary antibody. In this case, the secondary antibody is preferably labeled with a labeling substance.

Specifically, a solution containing an anti-GPC3 antibody is added to a support such as a plate to allow the anti-GPC3 antibody to be immobilized on the support. After the plate is washed, the plate is blocked with, for example, BSA in order to prevent any non-specific biding of proteins. The plate is washed again, and an assay sample is added to the plate. After incubation, the plate is washed and a primary anti-GPC3 antibody is added. After appropriate incubation, the plate is washed and a secondary antibody capable of specifically recognizing the primary antibody is added. After appropriate incubation, the plate is washed and the secondary antibody remaining on the plate is detected. The detection of the secondary antibody may be carried out according to the method described above.

In still another embodiment of the method according to the invention, GPC3 protein is detected by utilizing an agglutination reaction. In this method, GPC3 is detected using a support sensitized with an anti-GPC antibody. The support to be sensitized with an antibody may be of any type, as long as it is insoluble and does not cause any non-specific reaction with the antibody and is stable. Such a support include, for example, latex particles, bentonite, collodion, kaolin, and fixed sheep erythrocytes; preferably latex particles. The latex particles may include, for example, polystyrene latex particles, styrene-butadiene copolymer latex particles, polyvinyl-toluene latex particles. Preferred are polystyrene latex particles. The sensitized particles are mixed with a sample, and stirred for a predetermined period of time. The degree of agglutination of the particles becomes larger as the concentration of the GPC3 protein in the sample is higher. Therefore, GPC3 in the sample may be detected by visual observation for the agglutination of the particles. In addition, the turbidity due to the agglutination may be measured by a spectrophotometer or the like, whereby the protein in the sample may be detected.

In still another embodiment of the method according to the invention, GPC3 protein is detected by, for example, a biosensor utilizing the surface plasmon resonance phenomenon. The biosensor based on such a surface plasmon resonance phenomenon enables real-time detection of a protein-protein interaction indicated by a surface plasmon resonance signal by the use of a small amount of a unlabeled protein. For example, the binding of GPC3 protein to an anti-GPC3 antibody can be detected with a biosensor such as BIAcore (by Pharmacia). Specifically, an assay sample is contacted with a sensor chip with an anti-GPC3 antibody immobilized thereon, and the GPC3 protein bound to the anti-GPC3 antibody is detected as a change in the resonance signal.

The detection method of the invention can be automated by the use of various automatic detection devices, where a large amount of a sample can be detected at once.

The invention also provides a diagnostic agent or a kit for diagnosing cancer by detecting GPC3 protein in an assay sample, where the diagnostic agent or the kit contains the anti-GPC3 antibody. Where the diagnostic agent or the kit is based on ELISA, the agent or kit may contain a support for immobilizing the antibody, or the antibody may be previously immobilized on the support. Where the diagnostic agent or the kit is based on an agglutination method using a support such as latex, the agent or kit may contain a support with an antibody adsorbed thereon. The kit may optionally contain a blocking solution, a reaction solution, a reaction-stopping solution or a reagent for sample treatment.

All the contents of the patent references and other references explicitly referred to in this description are incorporated herein by reference. In addition, the entire contents of the specification and the drawings of Japanese Patent Application No. 2003-313187, which is the basic application for the priority of the present application, are incorporated herein by reference.

### EXAMPLES

The invention is described in more detail with reference to the following Examples. However, the technical scope of the invention should not be limited to those Examples.

### [Example 1] Detection of GPC3 by immunohistochemical staining of cholangiocarcinoma tissue

Cholangiocarcinoma tissue samples (five samples, formalin-fixed paraffin-embedded specimens) were subjected to immuno-histological staining using an anti-GPC antibody. The samples were stained using 1 µl/ml solution of an anti-human GPC3 antibody (Chugai Seiyaku, Genome Antibody Medicine Laboratory Section, Clone: M11F1, Class: IgG2b) as a primary antibody, and Biotinylated Anti-Mouse IgG (H+L) (Vector Laboratories Inc., BA-2000) as a secondary antibody. The coloration reaction was carried out using Vectaatain ABD-AP kit (Vector Laboratories Inc., AK-5000) and Red Alkaline Phosphatase Substrate Kit I (Vector Laboratories Inc., SK-5100).

In addition to the staining with anti-GPC3 antibody, all the samples were also examined by immunostaining with CD31 and vimentin to ensure the protein expression in the organs. Further, the positive reaction specificity of the staining was examined by immunostaining specimens from the adjacent tissue using a negative control antibody (IgG2b isotype control mouse monoclonal antibody) as a primary antibody.

Positive signal was observed for all the five samples of the cholangiocarcinoma tissue tumor cells tested for the immunostaining. In four out of five samples, the intensity of the positive signal in the tumor cells was stronger than that observed in the normal bile duct epithelial cells around the tumor cells; and in the rest one sample, the positive signal was at the same level for the two sites.

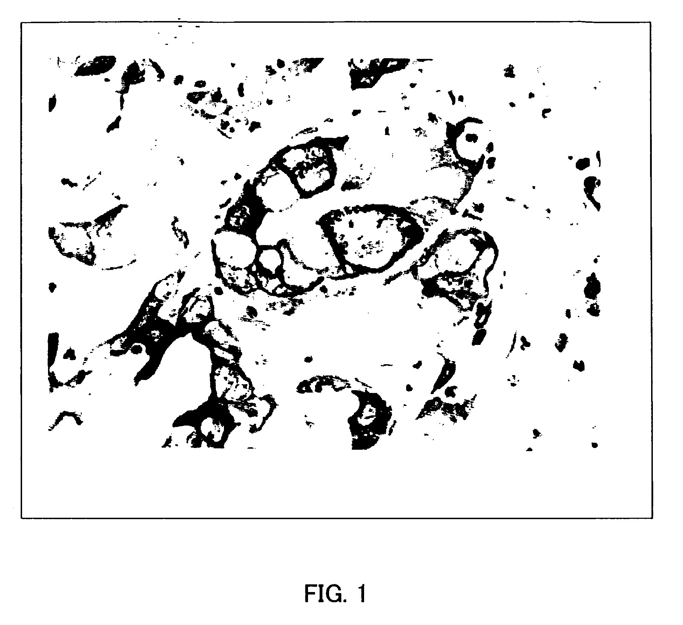

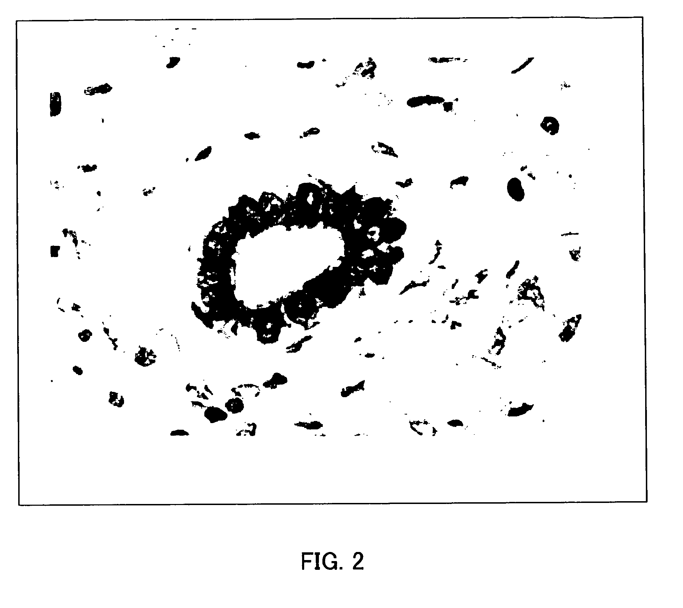

Regarding the intracellular localization of the positive signal, the signal was observed uniformly in the cell membrane of the cholangiocarcinoma cells, while the signal was observed locally in the bile duct inner lumen in the normal cells around the cholangiocarcinoma cells. Figs. 1 and 2 are photographs showing the result of immunostaining of cholangiocarcinoma cells and normal cells, respectively. Fig. 1; a strong positive signal is observed uniformly in the cell membrane. Fig. 2; the positive signal is localized on the bile duct inner lumen.

## Claims

1. A cholangiocarcinoma cell growth inhibitor comprising an anti-glypican-3 antibody.

2. The cholangiocarcinoma cell growth inhibitor as claimed in claim 1, wherein the anti-glypican-3 antibody has a cytotoxic activity.

3. The cholangiocarcinoma cell growth inhibitor as claimed in claim 2, wherein the cytotoxic activity is an antibody-dependent cytotoxic (ADCC) activity or a complement-dependent cytotoxic (CDC) activity.

4. The cholangiocarcinoma cell growth inhibitor as claimed in claim 1, wherein the anti-glypican-3 antibody is an antibody having a cytotoxic substance attached thereto.

5. The cholangiocarcinoma cell growth inhibitor as claimed in any one of claims 1 to 4, wherein the antibody is a monoclonal antibody.

6. The cholangiocarcinoma cell growth inhibitor as claimed in any one of claims 1 to 4, wherein the antibody is a humanized or chimera antibody.

7. A diagnostic agent for cholangiocarcinoma comprising an anti-glypican-3 antibody.

8. The diagnostic agent for cholangiocarcinoma as claimed in claim 7 comprising an anti-glypican-3 antibody immobilized on a support and an antibody labeled with a labeling substance.

9. The diagnostic agent for cholangiocarcinoma as claimed in claim 7 or 8, wherein the antibody is a monoclonal antibody.

10. A diagnostic kit for diagnosis of cholangiocarcinoma comprising an anti-glypican-3 antibody.

11. The diagnostic kit for diagnosis of cholangiocarcinoma as claimed in claim 10 comprising an anti-glypican-3 antibody immobilized on a support and an antibody labeled with a labeling substance.

12. A method for inhibiting the growth of cholangiocarcinoma cells comprising administering an anti-glypican-3 antibody to a patient suffered from cholangiocarcinoma.

13. A method for diagnosing cholangiocarcinoma comprising detecting a glypican-3 protein in an assay sample.
